# EUROPEAN PATENT APPLICATION

(11) **EP 4 137 144 A2**
(43) Date of publication of application: **22.02.2023**
(21) Application number: 21754903.9
(22) Date of filing: 12.04.2021
(51) Int. Cl.: A61K 38/17, A61P 31/12, A61P 37/00

(54) **PEPTIDE FOR THE TREATMENT OF CYTOKINE STORM SYNDROME**

(30) Priority: 13.04.2020 CU 20200026
(71) Applicant: Centro de Ingeniería Genética y Biotecnología, La Habana 11600 (CU)
(72) Inventor: DOMÍNGUEZ HORTA, María del Carmen, Cubanacán, Playa La Habana 11600 (CU); VENEGAS RODRIGUEZ, Rafael, Habana del Este La Habana 11700 (CU); MARTINEZ DONATO, Gillian, Siboney, Playa La Habana 11600 (CU); GUILLEN NIETO, Gerardo, Enrique, Playa La Habana 11600 (CU); HERNÁNDEZ CEDEÑO, Mabel, Marianao La Habana 11500 (CU); ORTEGA GONZÁLEZ, Lilia, María, Playa La Habana 11300 (CU); NODARSE CUNI, Hugo, La Habana 10600 (CU); LOPEZ ABAD, Cruz Matilde, Playa La Habana 11600 (CU); GARAY PEREZ, Hilda, Elisa, Playa La Habana 11600 (CU); UBIETA GÓMEZ, Raimundo, Playa La Habana 11600 (CU)
(74) Representative: V.O.
(86) International application number: PCT/CU2021/050001
(87) International publication number: WO 2021/209080

(57) **Abstract**

Pharmaceutical composition comprising the APL-type peptide identified as SEQ ID NO: 1, as an inhibitor of a state of hyperinflammation characterized by an increase in cytokines or interleukins and inflammatory molecules, which increase their concentration until reaching the state defined as "cytokine storm". The sucrose concentrations in said composition increase the biological activity of the peptide, facilitating its intravenous application. Said peptide in a pharmaceutical composition is useful for the manufacture of a drug to treat diseases related to hyperinflammation, such as Covid-19, dengue, macrophage activation syndrome, respiratory sepsis and chimeric antigen receptor T-Cell therapies, and acute respiratory distress syndrome. It also discloses a method for the treatment of said diseases, administering a therapeutically effective amount of the composition.

## Description

### Technical field

The present invention relates to the field of biomedicine and the pharmaceutical industry, in particular to a pharmaceutical composition for the treatment and prevention of cytokine storm syndrome. The composition comprises the peptide identified as SEQ ID NO: 1, and can be used in the treatment of acute respiratory distress syndrome, macrophage activation syndrome, respiratory sepsis, dengue, hepatic encephalopathy and pericarditis, among other diseases.

### State of the prior art

Within the individuals infected with the SARS-CoV-2 virus who develop the infectious disease Covid-19, a subgroup of patients has been described in whom a state of hyperinflammation is generated. In these patients, a "cytokine storm" is described, particularly of interleukins (IL), characterized by an increase in IL-2, IL-17, IL-6, TNFα, among others. This state of hyperinflammation leads to the death of patients (Mehta, et al. (2020). The Lancet, 395: 1033-1034).

On the other hand, in a retrospective study, which included 150 confirmed cases with Covid-19 in Wuhan, China, a significant increase in ferritin concentration was demonstrated in patients who did not survive (1297.6 ng/ml), compared to the levels of this protein in patients who survived the disease (614 ng/ml) (Rúan et al. (2020). Intensive Care Med; https://doi.org/10.1007/s00134-020-05991-x).

It is known that patients in serious and critical condition affected by Covid-19 develop acute respiratory distress syndrome. This syndrome is characterized by the presence of inflammatory events in the alveolus, which expand through the body's systemic circulation. In the course of this syndrome, pulmonary endothelial involvement occurs, with increased vascular permeability, and the traffic of cells and macromolecules to the alveolar space, where they inactivate the pulmonary surfactant, establishing the typical hyaline membranes (Janz and Ware (2014) Clin Chest Med; 35: 685-96). The epithelial barrier is also altered, and an inflammatory response is exacerbated with secretion of pro-inflammatory IL, such as TNFα, IL-1β, and IL-6, and other mediators of inflammation, with the subsequent activation of neutrophils, monocytes, macrophages, and the traffic of these molecules and cells to the alveolar spaces, increasing the initial involvement. The development of this hyperinflammation occurs in other diseases such as respiratory sepsis, macrophage activation syndrome, and sepsis in adults and neonates.

To date, there is no effective therapy for patients with Covid-19 disease. Therapies approved for the treatment of other diseases are being used, such as the administration of monoclonal antibodies against IL-1 (Anakinra), IL-6 (Tocilizumab), and Janus kinase protein inhibitors. However, these drugs have not been effective, and it are also immunosuppressive, so the use of these therapies to treat Covid-19 can lead to a worsening of the general condition of the patient, since viral persistence has been shown during course of the disease and even after clinical recovery of the patients. There is also no effective and safe cure for respiratory sepsis, macrophage activation syndrome, neonatal sepsis, as well as respiratory distress.

Therefore, it is of great interest to obtain drugs that are effective for the treatment of hyperinflammation and the "cytokine storm" that characterizes these pathologies.

### Explanation of the invention

The present invention resolves the aforementioned problem, by providing a pharmaceutical composition for the treatment and prevention of cytokine storm syndrome that comprises a concentration of 1.8 - 3.6 mg / mL of the peptide identified as SEQ ID NO: 1 and a sucrose concentration between 20-30 mg / mL, which surprisingly synergistically increases the biological activity of said peptide, in addition to being a determining factor for the induction of regulatory T cells, which control the magnitude of the inflammation. The composition of the invention is capable of reducing the hyperinflammation that characterizes the subgroup of patients with Covid-19 who progress to a severe stage, characterized by respiratory distress, as occurs in respiratory sepsis, macrophage activation syndrome, and neonatal and adult sepsis. The administration of the peptide identified as SEQ ID NO: 1 reduces the level of pro-inflammatory cytokines in patients with Covid-19 disease, without causing immunosuppression or serious adverse effects or those associated with peptide therapy.

The peptide identified as SEQ ID NO: 1 is derived from a region of the HSP60 protein, comprised between amino acids 83 to 109. HSP60 is highly immunogenic, both in healthy individuals and in patients with autoimmune diseases. However, the level of antibodies and T cells that this protein can induce is higher in patients with autoimmune diseases (de Jong et al (2009). Arthritis Rheum. 7 (60): 1966-1976). This protein has a great capacity to stimulate the pro-inflammatory reactivity of the innate immune system. HSP60 has also been found to induce the expression of IL-12 and IL-15 genes, cytokines involved in the induction of a Th1 phenotype. These facts suggest that HSP60 acts as a danger signal for the innate immune system (Habich et al (2005). J.Immunol. 3 (174): 1298-1305). On the other hand, peptides derived from HSP60 can also constitute a danger signal (Quintana and Cohen (2011). Trends Immunol. 2 (32): 89-95).

The peptide of SEQ ID NO: 1 contains several overlapping T-cell epitopes. It increases the frequency of Treg cells with the CD4⁺CD25^{high}Foxp3⁺ phenotype in *ex vivo* assays with peripheral blood mononuclear cells from rheumatoid arthritis patients. However, this is not the case with cells from healthy donors (Barbera et al (2016). Cell Stress. Chaperones 4 (21): 735-744).

The International patent applications No. PCT/CU2005/000008 and PCT/CU2009/000009 claim the peptide identified as SEQ ID NO: 1 and its use for the treatment of rheumatoid arthritis and Crohn's disease, colitis ulcerative and type I diabetes mellitus, respectively. All these pathologies are non-infectious diseases. Furthermore, patent application WO2019/129315 describe a very stable pharmaceutical composition containing the peptide of SEQ ID NO: 1, its administration causes the reduction of antibodies against citrullinated peptides and of other parameters closely associated with the citrullination of proteins. The peptide identified as SEQ ID NO: 1 is obtained by chemical synthesis. Through experiments such as those described in the present invention, those immunological mechanisms induced by the peptide that participate in the control of hyperinflammation can be evaluated. The pharmaceutical composition was well tolerated and had a high safety profile, with no evidence of immunosuppression. No serious adverse events were identified in any patient. For the purposes of this invention, cytokine storm syndrome is understood as the increase in pro-inflammatory cytokines, where concentrations are reached that exceed normal levels. These pro-inflammatory cytokines are IL-6, TNFα, IL-1, IL-17, among others. It also refers to the increase in other cytokines, such as IL-10, IL-8, and IL-5. In addition, it includes the increase in biomarkers of inflammation, such as C-reactive protein, fibrinogen, D-dimer, hepatic transaminases, lactate dehydrogenase and ferritin. The increase in these molecules associated with the cytokine storm occurs in patients affected by inflammatory pathologies such as: acute respiratory distress, macrophage activation syndrome, respiratory sepsis, dengue, hepatic encephalopathy and pericarditis. From a clinical point of view, sustained fever, weakness, fatigue and nausea can be evidenced in patients.

In another aspect, the invention discloses the use of a pharmaceutical composition comprising the peptide identified as SEQ ID NO: 1 in the manufacture of a medicament for the treatment and prevention of cytokine storm syndrome. Surprisingly, treatment with a composition comprising said peptide allows patients with Covid-19 to be the withdrawn from mechanical ventilation, based on the improvement of interstitial pneumonia, the decrease in the reactants of the acute phase of inflammation between 24 and 48 hours, and the reduction of biomarkers of inflammation. The rapid decline in C-reactive protein, in just 24 hours, is an unexpected finding. Previously, in patients with rheumatoid arthritis who were treated with this peptide, a significant decrease in C-reactive protein could not be demonstrated (Prada, et al (2018). J Clin Trials 1 (8): 1-11).

The present invention demonstrates the ability of the peptide identified as SEQ ID NO: 1, administered intravenously, to reduce respiratory distress, hyperinflammation, and reactants of the acute phase of inflammation, and the level of pro-inflammatory cytokines in patients with Covid-19. Surprisingly, these results were reproduced in patients with respiratory sepsis who presented with the respiratory distress syndrome.

In a particular embodiment, the pharmaceutical composition comprising the peptide identified as SEQ ID NO: 1 is used in the manufacture of a medicament for the treatment and prevention of cytokine storm syndrome that manifests as acute respiratory distress syndrome, macrophage activation syndrome, respiratory sepsis in adults or neonates, systemic inflammatory response syndrome, venous or arterial thromboembolism, acute elevation of ferritin levels, severe pericarditis, acute elevation of hepatic transaminases causing hepatic encephalopathy or disease associated with chimeric antigen receptor T-cell therapies. Among the pathologies that are treated with the pharmaceutical composition that comprises the peptide identified as SEQ ID NO: 1 is the cytokine storm syndrome that underlies the systemic inflammatory response syndrome, which occurs in patients after burns or severe trauma or in acute inflammatory diseases. In one embodiment of the invention, the drug is administered intravenously.

In one embodiment of the invention, the medicament is used for the treatment and prevention of cytokine storm syndrome caused by an infectious disease. In a particular embodiment, said infectious disease is Covid-19 or dengue. The object of the present invention is a method for the treatment or prevention of cytokine storm syndrome that comprises the administration of a pharmaceutically effective amount of a pharmaceutical composition comprising the peptide identified as SEQ ID NO: 1, to an individual in need thereof. In a particular embodiment, the cytokine storm syndrome manifests as acute respiratory distress syndrome, macrophage activation syndrome, respiratory sepsis in adults or neonates, systemic inflammatory response syndrome, venous or arterial thromboembolism, acute elevation of ferritin, severe pericarditis, acute elevation of the hepatic transaminases that cause hepatic encephalopathy or disease associated with chimeric antigen receptor T-cell therapy. In one embodiment, the cytokine storm that is treated or prevented by the method of the invention is caused by an infectious disease. In a preferred embodiment the method of the invention is useful for the treatment of the infectious disease as Covid-19 or dengue. In an embodiment of the invention, in said method the composition is administered intravenously.

The administration to patients who need it of the pharmaceutical composition comprising the peptide identified as SEQ ID NO: 1 decreases events associated with respiratory distress. This distress causes patients to need:
- Oxygen therapy to maintain oxygen saturation (SO₂) above 93%
- Oxygen saturation greater than 3 percentage points or decrease in arterial partial pressure of oxygen (PaO₂) greater than 10%, with stable fraction of inspired oxygen (FiO₂) in the last 24 hours
- Increased FiO₂ in order to maintain a stable SO2 or new need for mechanical ventilation in the last 24 hours
- Increase in the number and/or extension of the pulmonary areas of consolidation.

In addition, the administration of the composition of the invention is useful for the treatment of patients affected with the infectious disease Covid-19, when said patients need oxygen therapy of no less than 6 L/min plus one of the following conditions:
- Wheezing or breathy speech
- Respiratory rate greater than 22 breaths/min with oxygen therapy at 6 L/min
- PaO₂: Less than 65 mm Hg
- Worsening of the radiological image
- Fever greater than or equal to 39°C
- Reduction in baseline values of hemoglobin, platelets and leukocytes (hemoglobin ≤9.2 gm/dl, leukocytes ≤5000 /mm³, platelets ≤110,000 /mm
- Decreased erythrocyte sedimentation rate in discordance with C-reactive protein that is increased or unchanged
- Increase in baseline value of triglycerides above 3 mmol/L
- Increase in the baseline value of ferritin from 500 ng/ml or absolute value of ferritin greater than or equal to 2000 ng/ml
- Aspartate-aminotransferase transaminase greater than or equal to 30 IU/L
- Increase in D-dimer
- Fibrinogen less than 2.5 g/L
- Appearance of neurological manifestations

The composition of the invention is also useful for the treatment of patients with Covid-19 who enter a serious state without the need for oxygen therapy, but who present an increase in the initial value of ferritin from 500 ng/ml or absolute value of ferritin greater than or equal to 2000 ng/ml, with progressive radiological signs of multifocal interstitial pneumonia.

The composition of the invention is also useful for the treatment of diseases characterized by a state of acute hyperinflammation such as respiratory sepsis in adults or neonates; and for diseases that develop respiratory distress syndrome, as well as for said syndrome caused by mechanical and physical causes.

### Brief description of the figures

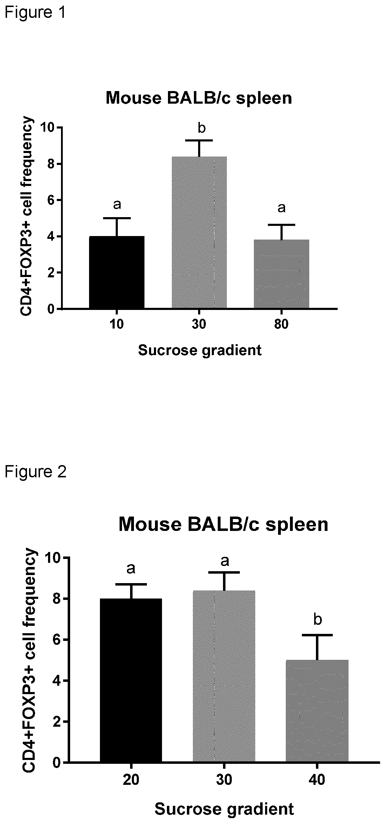

**Figure 1A.** Frequency of T cells quantified by flow cytometry that express the CD4 + Foxp3 + phenotype, isolated from the spleen of BALB/c mice, after being inoculated with the pharmaceutical composition of the peptide identified as SEQ ID NO: 1, where sucrose was added at a concentration10 mg/mL (10), 30 mg/mL (30) and 80 mg/mL (80). The Kruskal-Wallis and Dunn statistical tests were used for data analysis. Different letters indicate statistically significant differences (P <0.05).
**Figure 1B.** Frequency of T cells quantified by flow cytometry that express the CD4 + Foxp3 + phenotype, isolated from the spleen of BALB / c mice, after being inoculated with the pharmaceutical composition of the peptide identified as SEQ ID NO: 1, where sucrose was added at a concentration of 20 mg/mL (20), 30 mg/mL (30) and 40 mg/mL (40). The Kruskal-Wallis and Dunn statistical tests were used for data analysis. Different letters indicate statistically significant differences (P <0.05).

### Detailed exposition of embodiments / Embodiment examples Example 1. Treatment of patients with Covid-19 disease with the pharmaceutical composition comprising the peptide identified as SEQ ID NO: 1.

It is exemplified with ten patients admitted to the Intensive Care Unit of the Luis Diaz Soto Military Hospital, affected with the infectious disease Covid-19. These patients received medical attention according to the protocol established by the Cuban Ministry of Public Health for the treatment of this disease. These patients developed acute respiratory distress syndrome, and on the same day they presented it, they underwent mechanical artificial ventilation/ airway pressure release ventilation (VAM APRV). After receiving informed consent, as established by the Cuban regulatory authority and the Ethics and Review Committee of said hospital, the patients received the pharmaceutical composition comprising the peptide identified as SEQ ID NO: 1. Said peptide was obtained by synthesis chemistry, by a method well known in this field of art.

The pharmaceutical composition was administered intravenously, once every 12 hours. In eight of the ten patients, 1 mg of the peptide was administered per patient in each application. The Patients with code 5 and 6 received 2 mg every 12 hour since they had cancer among the comorbidities. The characteristics of the patients, as well as the days of VAM APRV and administration of the peptide are reflected in Table 1.

**Table 1. Characteristics of the patients treated with the pharmaceutical composition comprising the peptide identified as SEQ ID NO: 1.**

| **Patients** | **Age, (years)** | **Comorbidities** | **Days of VAM APRV** | **Days of therapy with peptide** |
|---|---|---|---|---|
| 1 | 70 | Arrhythmias | 7 | 10 |
| 2 | 42 | AHT smoking | 5 | 9 |
| 3 | 64 | AHT | 8 | 11 |
| 4 | 60 | AHT | 7 | 9 |
| 5 | 42 | Obesity | 19 | 22 |
| | | Cushing's syndrome | | |
| | | Adrenal gland tumor | | |
| 6 | 79 | Diabetes mellitus | 5 | 8 |
| | | Bladder cancer | | |
| 7 | 80 | - | 4 | 7 |
| 8 | 84 | AHT | 3 | 6 |
| | | Bronchial asthma | | |
| 9 | 87 | Bronchial asthma | 7 | 5 |
| 10 | 68 | AHT | 7 | 6 |

| | | | | |
|---|---|---|---|---|
| AHT: Arterial hypertension. VAM APRV: Mechanical artificial ventilation/ airway pressure release ventilation. | | | | |

As can be seen in the table, the ten patients with Covid-19 disease, who were treated with the peptide had risk factors that predispose to a serious or critical state of the disease. Only one patient did not present comorbidities, but his advanced age (80 years old), this constitutes a risk factor. In particular, the patient identified with no. 5 presented a deep vein thrombosis. However, the clinical evolution of the ten patients, when the peptide was administered, was favorable. At the time of extubation, the acute respiratory distress syndrome is considered to end. These results demonstrate that the treatment with peptide was effective for the acute respiratory distress syndrome. The disappearance of this syndrome in the patients coincided with their clinical and radiological improvement. The patient who presented with deep vein thrombosis also evolved favorably. These patients did not present any symptoms of immunosuppression. Before therapy they presented lymphopenia, a characteristic sign of patients with Covid-19. As a result of the treatment with the peptide, the levels of circulating lymphocytes gradually recovered to normality.

The specialized literature reports that 85% -90% of patients with acute respiratory distress syndrome do not survive. In Cuba, among the patients confirmed with SAR-COV-2 who transited to a critical or severe stage, there was a fatality rate of 68.6% in individuals who were not treated with the pharmaceutical composition that comprises the peptide identified as SEQ ID NO: 1. In contrast, the fatality rate was 13.0% in critical or seriously ill patients, confirmed with SAR-COV-2, who were treated with the pharmaceutical composition comprising the peptide identified as SEQ ID NO: 1.

### Example 2. Administration of the pharmaceutical composition comprising the peptide identified as SEQ ID NO: 1 to prevent the establishment of respiratory distress syndrome.

Seriously ill and critically ill patients with Covid-19 develop respiratory distress syndrome, which can lead to death. Surprisingly, the administration of the pharmaceutical composition comprising the peptide identified as SEQ ID NO: 1 prevented the development of an acute respiratory distress syndrome. The peptide was administered at an early stage, after the first signs (progressive radiological indicators of multifocal interstitial pneumonia, elevated ferritin (2 times above normal value), pulse oximetry below 94 oxygen saturation). The administration of said peptide therefore prevented the need for artificial ventilation. In this way, the probable evolution to nosocomial sepsis was avoided. It is exemplified with a 42-year-old female patient, affected with the Covid-19 disease, admitted to the intermediate therapy room of the Hospital of the "Pedro Kouri" Institute of Tropical Medicine. This patient received medical attention, according to the protocol established by the Cuban Ministry of Public Health for the treatment of this disease, but he presented sudden dyspnea, with a fever of 39 °C and a ferritin concentration of 700 ng/mL. With informed consent, the pharmaceutical composition containing the peptide identified as SEQ ID NO: 1 was administered, intravenously, in a dose of 1 mg, every 12 hours. After 2 hours, the dyspnea condition disappeared and the body temperature decreased. The patient did not develop acute respiratory distress syndrome, and the ferritin concentration decreased below 500 ng/mL at 72 hours.

In addition, three other patients in serious condition were treated, the demographic characteristics and comorbidities are presented in Table 2. These patients were admitted to the intermediate therapy unit from the Luis Diaz Soto Central Military Hospital.

**Table 2. Age and comorbidities of seriously ill patients treated with the pharmaceutical composition comprising the peptide identified as SEQ ID NO: 1.**

| **Patients** | **Age (years)** | **Comorbidity** |
|---|---|---|
| 1 | 19 | - |
| 2 | 76 | AHT |
| | | Diabetes mellitis |
| | | Cardiac ischemia |
| 3 | 96 | Diabetes mellitis |
| | | Cardiac ischemia |

| | | |
|---|---|---|
| AHT: Arterial hypertension. | | |

These patients also presented the first warning signs, such as: sustained fever above 38 °C, dyspnea requiring oxygen therapy by mask or nasal fork, to maintain an oxygen saturation above 93. They also presented polypnea greater than 25 ventilations per minute and required oxygen therapy by mask or nasal fork, to maintain oxygen saturation above 93.

The administration of the pharmaceutical composition containing the peptide identified as SEQ ID NO: 1, was by intravenous route, in a dose of 1 mg, every 24 hours. After 2 hours of treatment, the dyspnea disappeared, and the body temperature decreased. The patients did not develop acute respiratory distress syndrome. The ferritin concentration dropped below 200 ng / mL after 72 hours. These results demonstrate that the treatment of patients with Covid-19 disease with the pharmaceutical composition containing the peptide identified as SEQ ID NO: 1, before they develop acute respiratory distress syndrome, avoids the use of mechanical ventilation for these patients.

### Example 3. Decrease in C-reactive protein in patients with Covid-19 disease treated with the pharmaceutical composition comprising the peptide identified as SEQ ID NO: 1.

C-reactive protein is one of the most used markers for inflammatory processes; it is considered one of the reactants of the acute phase of inflammation. The pharmaceutical composition comprising the peptide identified as SEQ ID NO: 1 was administered intravenously. Eight of the ten patients affected with Covid-19, whose treatment is described in Example 1, received 1 mg of the peptide every 12 hours. Patients with code 5 and 6 who had cancer between comorbidities received 2 mg of the peptide every 12 hours. Unexpectedly, treatment with the peptide of SEQ ID NO: 1 decreased C-reactive protein levels, markedly, in treated patients. These results are shown in Table 3.

**Table 3. C-reactive protein concentration (mg/mL) in patients treated with the peptide identified as SEQ ID NO: 1.**

| **Patients** | **At the beginning of treatment** | **After treatment** | | |
|---|---|---|---|---|
| | | **Day 1** | **Day 3** | **Day 4** |
| 1 | 131 | 130 | 86 | 24 |
| 2 | 276 | 157 | 147 | 75 |
| 3 | 195 | 108 | 86 | 55 |
| 4 | 60 | 49 | 17 | 4 |
| 5 | 494 | 141 | 51 | 29 |
| 6 | 195 | 165 | 86 | 55 |
| 7 | 299 | 198 | 176 | 45 |
| 8 | 140 | 135 | - | 37 |
| 9 | 232 | 195 | 165 | 86 |
| 10 | 215 | 200 | 167 | 118 |

| | | | | |
|---|---|---|---|---|
| -: Not quantified. | | | | |

The decrease in C-reactive protein during the treatment of these patients is a direct indication that the peptide of SEQ ID NO: 1 reduces inflammation, and this result is according with the disappearance of acute respiratory distress syndrome, as well as with the clinical and radiological improvement of the patients.

The results obtained in this study are surprising. During a clinical trial, in patients with rheumatoid arthritis treated with the same peptide, no decrease in this reactant was found in the acute phase of inflammation.

### Example 4. Decrease of pro-inflammatory cytokines in patients with Covid-19 disease treated with the pharmaceutical composition comprising the peptide identified as SEQ ID NO: 1.

In this study, the levels of the pro-inflammatory cytokines IL-6, IL-17, IL-1, IFNγ and TNFα were determined in patients with Covid-19 disease treated with the composition comprising the peptide identified as SEQ ID NO: 1. The patients were in critical condition. The treatment of these patients was described in Example 1.

Said cytokines were quantified in the sera from the patients identified with the numbers 1, 2, 3, 4, 7 and 8, which appear in Table 1, Example 1. For the determination of the cytokines in the serum samples, the virus was inactivated, under strict biosecurity measures. Quantification was carried out just before applying the first dose of the peptide, and at 48, 72 and 96 hours after starting treatment with the peptide.

All cytokine determinations were carried out using an ELISA (enzyme-linked immunosorbent assay) specific for each one, following the manufacturer's recommendations (Quantikine^{®} R&D Systems, USA). Briefly, 50 µL of the specified diluent for each cytokine was added to the wells, followed by 200 µL of the standard curve, sample, or control. The plate was incubated for 2 h at room temperature, and then the contents of each well were discarded. Then three washes were performed with the 1X wash solution, 200 µL of the conjugated antibody was added to each well, and incubated for 1 hr at room temperature. The wash was repeated and 200 µL of the substrate solution was added to each well. The plate was incubated for 20 min at room temperature protected from light, 50 µL of the stop solution was added to each well, and the optical density was determined at 450 nm in a microplate reader (Thermo Scientific Multiskan Go, Finland). A second reading was made at 570 nm, and the values obtained were subtracted from the 450 nm reading, to correct the optical imperfections on the plate. The determination of the concentration of each cytokine was carried out in triplicate. The results obtained for IL-6 are shown in Table 4.

**Table 4. IL-6 cytokine levels (pg/mL) found in patients treated with the peptide.**

| **Patients** | **Before administration of the peptide** | **Time after peptide administration** | | |
|---|---|---|---|---|
| | | **48 h** | **72 h** | **96 h** |
| 1 | 177,56 | 14,45 | 20,03 | 1,71 |
| 2 | 61,045 | 12,08 | 17,965 | 5,715 |
| 3 | 61,245 | 0,68 | 0,68 | 0,98 |
| 4 | 154,95 | 202,52 | 1,95 | 51,57 |
| 7 | 145,99 | 11,33 | 14,12 | 19,76 |
| 8 | 82,36 | 1,99 | - | 3,43 |

As can be seen in the table, there was a decrease in the levels of the cytokine IL-6, since the critically ill patients received the peptide of SEQ ID NO: 1. This is a cytokine that increases markedly in inflammatory processes. It has been described that patients with Covid-19 disease who transition to a severe stage have elevated this cytokine, in contrast to patients who present mild symptoms of the disease. Similarly, a decrease in IL-17, IL-1, IFNγ and TNFα was found in these patients.

These results confirm that treatment with the peptide reduces hyperinflammation, and the cytokine storm, developed by patients with Covid-19 disease who are transitioning to a severe stage.

### Example 5. Treatment of a patient with Covid-19 disease who presented high concentrations of ferritin without respiratory distress with the pharmaceutical composition comprising the peptide identified as SEQ ID NO: 1.

The patient treated was 66 years old, female, with a history of type 2 diabetes mellitus, exogenous obesity, grade 4 fatty liver disease. He was admitted, due to a diagnosis of COVID-19, he presented digestive symptoms such as dyspepsia, nausea, occasional diarrhea, for which prokinetics and anti-emetics were indicated. Besides , the patients presented high concentrations of ferritin, above 1500 ng/mL, and presented an increase in the erythrocyte sedimentation rate with a fall in hemoglobin.

Due to this clinical condition, the specialists indicated treatment with the pharmaceutical composition that comprises the peptide identified as SEQ ID NO: 1, at a dose of 1 mg, every 12 hours. Surprisingly, after 96 hours of treatment, the ferritin values were reduced to a concentration of 350 ng/mL.

Treatment with the pharmaceutical composition comprising the peptide identified as SEQ ID NO: 1 continued for a further 72 hours.

Upon completion of the treatment, the ferritin concentration normalized, and the patient did not present any symptoms.

These results indicate that treatment with the pharmaceutical composition comprising the peptide identified as SEQ ID NO: 1, is capable of reducing the concentration of ferritin, when it increases due to a viral infection.

### Example 6. Treatment of a patient with pericarditis of viral etiology with the pharmaceutical composition comprising the peptide identified as SEQ ID NO: 1.

The treated patient was 48 years old, confirmed with Covid-19, and admitted to the Holguin Military Hospital. After two days of admission, the patient presented a fever of 38°C and two diarrheal stools, and was diagnosed with pericarditis secondary to myocarditis, of viral etiology (Covid-19), without hemodynamic repercussions. The patient did not present respiratory distress. Treatment was started with the pharmaceutical composition comprising the peptide identified as SEQ ID NO: 1, at a dose of 1 mg every 12 hours.

Surprisingly, after 24 hours of treatment, the electrocardiogram showed improvement, and at 96 hours of treatment the patient was asymptomatic, with significant clinical improvement. These results confirmed that the pharmaceutical composition comprising the peptide identified as SEQ ID NO: 1 reduces the inflammation that occurs in pathologies such as pericarditis secondary to myocarditis, without hemodynamic repercussions of viral etiology.

### Example 7. Treatment of a patient with Covid-19 hepatic encephalopathy with the pharmaceutical composition comprising the peptide identified as SEQ ID NO: 1.

This patient is female, 55 years old, Caucasian, with a pathological history of arterial hypertension. Said patient was admitted at the Luis Diaz Soto hospital, in the isolation room, internal medicine service, being positive for the coronavirus that causes the Covid-19 disease. Therefore, she began treatment for this disease, according to the protocol established in the country. During the admission she was asymptomatic until the tenth day. She said that day she presented episodes of anxiety or nervousness, associated with high blood pressure figures (160/90 mm Hg). She was evaluated by specialists in psychiatry, and complementary laboratory tests were performed. The patient did not present respiratory distress. The psychiatrist made the diagnosis of anxiety syndrome, with elements of a psychopathic syndrome. The patient worsened her clinical sings on day 11, she presented vomiting, high blood pressure figures, a tendency to tachycardia, without sphincter relaxation, so he was transferred to the intensive care unit. Hemogasometry showed the existence of severe hyponatremia (Na concentration 105 mmol/L). The Computed Axial Tomography did not show cranioencephalic alterations, and the complementary tests indicated markedly elevated liver enzymes, as well as a leukocytosis. Based on these results, the patient was diagnosed with hepatic encephalopathy. All the hepatotoxic medications were suspended from the patient, dietary changes were made, the gradual replacement of sodium was continued, and treatment with the pharmaceutical composition that comprises the peptide identified as SEQ ID NO: 1 was started, at a rate of 1 mg every 12 hours, intravenously. Daily clinical follow-up with complementary laboratory tests was carried out. Surprisingly, after 72 hours of treatment with the pharmaceutical composition that comprises the peptide identified as SEQ ID NO: 1, the evolution was favorable, with a decrease in liver enzymes, a progressive decrease in creatinine kinase and nitrogen compounds in the blood, as well as the normalization of the sodium concentration. At 96 hours of treatment, the clinical characteristics corresponding to a hepatic encephalopathy was completely reversed, the treatment with the pharmaceutical composition comprising the peptide was maintained for three more days. These results confirmed that the administration of the pharmaceutical composition comprising the peptide identified as SEQ ID NO: 1 reduces the inflammation that occurs in pathologies such as hepatic encephalopathy.

### Example 8. Treatment of a dengue patient with the pharmaceutical composition comprising the peptide identified as SEQ ID NO: 1.

A 19-year-old female patient who came to the Luis Diaz Soto hospital due to a fever of 38.5 °C, for more than 72 hours, and an intense holocranial headache. Also, the patient had retroocular pain associated with a discrete conjunctival injection. Based on these symptoms, the patient was admitted to the hospital with suspected dengue.

Physical examination of this patient revealed hyperthermia of 39.9 °C, blood pressure of 90/60 mm Hg, tachycardia, and mild distal coldness. Upon inspection of the skin, the doctor detected petechiae in the region of the forearms and abdomen, with a positive loop test. The patient was treated therapeutically with volume support at 30 mL/Kg of body weight, for 24 hours.

After 24 hours of hospital admission, the patient began with abdominal pain and vomiting. Complementary tests indicated leukopenia (2.9 × 10⁹ total leukocytes), erythrocyte sedimentation rate of 95 mm/h, C-reactive protein 150 mg/mL, Lactate Dehydrogenase 918 U/L, Glutamic Oxalacetic Transaminase 93 IU/L, Glutamic Pyruvic Transaminase 58 IU/L. Abdominal ultrasound indicated the presence of free fluid in all four angles, of moderate intensity. The gallbladder presented thickened walls and perivesicular edema, without lithiasis. Supportive therapeutic measures were applied to the patient, which included crystalloid solutions at a rate of 35-45 mL/kg of weight, for 24 hours.

The torpid evolution of the patient and the poor response to therapeutic measures based on fluid replacement indicated to medical team that the patient developed macrophage activation syndrome, secondary to infection by the virus that causes dengue. This diagnostic was verified by the values of the laboratory parameters associated with this syndrome, ferritin: 1893 mg/L; fibrinogen: 2.31 g/L; C-reactive protein: 150 mg/mL; erythrocyte sedimentation: 53 mm/h.

Based on these results, the patient was transferred to the intensive care unit, and the pharmaceutical composition comprising the peptide identified as SEQ ID NO: 1 was administered intravenously at a rate of 1 mg every 12 hours. The administration of said composition had the objective of modulating the immune response, and thus controlling the cytokine storm, which, according to the clinical characteristics and laboratory parameters were developments in this patient. After 48 hours of treatment, a noticeable improvement was observed in the patient, since abdominal pain, arterial hypotension and distal coldness disappeared. The patient began taking food. After 72 hours, continued the clinical and radiological improvement of the patient, from which the frequency of administration of the pharmaceutical composition comprising the peptide was reduced to 1 mg every 24 hours.

After the seventh day of treatment, the patient was clinically and radiologically asymptomatic. Dengue virus infection was confirmed, as IgM was positive in the serological test. All laboratory parameters were normalized, and the patient was discharged from the hospital.

These results confirmed those outlined in the previous examples, this example confirms that the pharmaceutical composition comprising the peptide identified as SEQ ID NO: 1 controls the cytokine storm, which may be linked to hemophagocytic syndrome that may be associated with various pathologies, such as dengue which are transitioning to a severe stage of the disease.

### Example 9. Effects of the sucrose concentration on the pharmacological potentiation of the peptide of SEQ ID NO: 1 for the reduction of inflammation.

In order to use the peptide identified as SEQ ID NO: 1, as an inductor of molecular mechanisms that reduce hyperinflammation in patients with COVID-19 and other pathologies, we studied the influence of sugar sucrose on the composition, in a range of concentrations, to select the optimal concentration of this sugar, associated with the induction of regulatory T cells, when the formulation is applied intravenously. For the preparation of the composition, 1.8mg/mL - 3.5mg/mL of the peptide identified as SEQ ID NO: 1 were used and it was dissolved in sodium acetate buffer, pH 4; to which the sucrose sugar was added in different concentrations, as described later.

Male mice of the BALB / c line were randomly separated into three groups of 10 mice each. The mice separated by group were inoculated intravenously, as follows:
- Group 1: pharmaceutical composition of the peptide identified as SEQ ID NO: 1, where sucrose was added at a concentration of 10 mg/mL.
- Group 2: pharmaceutical composition of the peptide identified as SEQ ID NO: 1, where sucrose was added at a concentration of 30 mg/mL.
- Group 3: pharmaceutical composition of the peptide identified as SEQ ID NO: 1, where sucrose was added at a concentration of 80 mg/mL.

Four days later, the mice received a second dose under identical conditions. The 10 animals per group were sacrificed on day 8 after receiving the first inoculation.

The spleen cells of the mice were preincubated with Mab 2.4G2 (*eBiosciences*, USA), to block non-specific binding to FC receptors. Next, the cells were labeled in PBS containing three percent calf serum and saturating amounts of the following mAbs (*eBiosciences*, USA): APC-Cy7-anti-CD4 conjugate (clone RM4-5) and APC-anti-CD25 conjugate ( PC61). The Foxp3 transcriptional factor was evaluated through the use of an anti-Foxp3 mAb conjugated to PE (FJK-16s; *eBioscience*, USA), following the manufacturers' recommendations. An isotype-irrelevant mAb (rat PE-Cy5 IgG2a; *eBioscience*, USA) was used as a control. The region corresponding to the lymphocytes was framed according to their distribution characteristics in the flow cytometry histograms on an LSR-II^{™} cytometer. The analyzes were carried out using the *FlowJo*^{®} program (*Tree Star*).

As can be seen in this Figure 1A, the mice inoculated with the formulation containing the peptide identified as SEQ ID NO: 1 and sucrose at a concentration of 30 mg/mL, significantly increased the percentage of regulatory T cells, which shows a synergistic effect between those components of the composition.

In order to determine whether sucrose at this concentration is decisive to induce the frequency of regulatory T cells, we conducted an experiment under conditions very similar to the previous one, but narrowing the concentration range of said sugar.

The mice were randomly separated into three groups of 10 mice each. The mice separated by group were inoculated intravenously, as follows:
- Group 1: pharmaceutical composition of the peptide identified as SEQ ID NO: 1, where sucrose was added at a concentration of 20 mg/mL.
- Group 2: pharmaceutical composition of the peptide identified as SEQ ID NO: 1, where sucrose was added at a concentration of 30 mg/mL.
- Group 3: pharmaceutical composition of the peptide identified as SEQ ID NO: 1, where sucrose was added at a concentration of 40 mg/mL.

Surprisingly, the mice treated with these concentrations of sucrose between 20-40 mg/mL showed a significant increase in the percentage of regulatory T cells compared to the groups treated with 10-80 mg/mL in the previous experiment, observing a synergistic effect between the components of the composition, demonstrating a greater effect in the groups of mice treated with the pharmaceutical composition of the peptide identified as SEQ ID NO: 1, where sucrose was added in concentrations of 20 and 30 mg/mL (Figure 1B).

These results allow us to conclude that the addition of sucrose in concentrations in the range of 20-30 mg/mL is a determining factor for the induction of regulatory T cells, which control the magnitude of inflammation, this formulation applied intravenously has the ability to control pathologies characterized by hyperinflammation.

## Claims

1. Pharmaceutical composition for the treatment and prevention of cytokine storm syndrome comprising the peptide identified as SEQ ID No. 1 and a pharmaceutically acceptable excipient.

2. Pharmaceutical composition of claim 1 **characterized by** comprises the peptide identified as SEQ ID NO: 1 in a concentration range between 1.8 mg/mL - 3.6 mg/mL and sucrose in a concentration between 20-30 mg/mL.

3. The composition of claim 1 **characterized in that** it is applied by intravenous route.

4. Use of a pharmaceutical composition comprising the peptide identified as SEQ ID No. 1 for the manufacture of a medicament for the treatment and prevention of cytokine storm syndrome.

5. The use of claim 4 where the cytokine storm syndrome manifests as acute respiratory distress syndrome, macrophage activation syndrome, respiratory sepsis in adults or neonates, systemic inflammatory response syndrome, venous or arterial thromboembolism, acute elevation of ferritin levels, severe pericarditis, acute elevation of liver transaminases causing hepatic encephalopathy, or disease associated with chimeric antigen receptor T-cell therapies.

6. The use of claim 4 wherein the composition is administered by intravenous route.

7. The use of claim 4 wherein the cytokine storm syndrome is caused by an infectious disease.

8. The use of claim 7 where the infectious disease is Covid-19 or dengue.

9. Method for the treatment or prevention of cytokine storm syndrome comprising the administration of a pharmaceutically effective amount of a pharmaceutical composition comprising the peptide identified as SEQ ID No. 1.

10. The method of claim 9 wherein the cytokine storm syndrome manifests as acute respiratory distress syndrome, macrophage activation syndrome, respiratory sepsis in adults or neonates, systemic inflammatory response syndrome, venous or arterial thromboembolism, acute elevation of ferritin levels, severe pericarditis, acute elevation of liver transaminases causing hepatic encephalopathy, or disease associated with chimeric antigen receptor T-cell therapies.

11. The method of claim 9 wherein the composition is administered by intravenous route.

12. The method of claim 9 wherein the cytokine storm syndrome is caused by an infectious disease.

13. The method of claim 12 where the infectious disease is Covid-19 or dengue
